# EUROPEAN PATENT APPLICATION

(11) **EP 1 132 399 A1**
(43) Date of publication of application: **12.09.2001**
(21) Application number: 00200826.6
(22) Date of filing: 08.03.2000
(51) Int. Cl.: C07K 14/37, A61K 38/16

(54) **Mannoproteins or equivalents thereof for use in modulating neutrophil migration**

(71) Applicant: Universitair Medisch Centrum Utrecht, 3584 CX Utrecht (NL)
(72) Inventor: HOEPELMAN, Ilja Mohandas, 3583 EC Utrecht (NL)
(74) Representative: Ottevangers, Sietse Ulbe

(57) **Abstract**

The present invention is among others concerned with suppressing neutrophil migration and the prevention of damage from neutrophil migration. Provided is a proteinaceous substance derivable from *Cryptococcus neoformans* and capable of interfering with neutrophil migration in a mammalian host. Also provided are functional equivalents of such a proteinaceous substance and the use of this substance and/or equivalent in the preparation of a medicament.

## Description

The present invention relates to the field of inflammatory diseases, in particular diseases involving neutrophil migration. A number of serious illnesses involve neutrophil transmigration at one stage or the other. Such diseases include among others respiratory diseases as seen in Adult Respiratory Distress Syndrome (ARDS), acute traumas (especially trauma to the brain), infections where neutrophils invade (e.g. Meningitis, peritonitis), Reperfusion ischemia (Stroke, heart attack, cardiopulmonary bypass surgery, spinal cord injury), Skin diseases (contact dermatitis and psoriasis) and autoimmune diseases (such as rheumatoid arthritis, inflammatory bowel diseases such ulcerative colitis and Crohn's disease).
A very important disease for which no successful drug has been developed sofar is bacterial meningitis.

Bacterial meningitis is a serious illness affecting annually in the United States about 3 individuals per 100,000 (1). The case fatality is estimated at 25% and a substantial percentage of the survivors will suffer permanent neurological impairment (2). Typically, a patient presents with serious clinical symptoms and a high leukocyte count in their cerebrospinal fluid (CSF; 3,4). Extensive literature evidence suggests that these - mainly neutrophil - leukocytes play an important adverse role in the pathogenesis of neurological damage (5 - 8).

It has been demonstrated that a high influx of neutrophil leukocytes into the central nervous system (CNS) correlates with an unfavorable prognosis for neurological recovery (9, 10). Therefore, several attempts have been made to reduce the inflammatory response (11). So far, the only studies published with adjuvant anti-inflammatory treatment in human bacterial meningitis, are clinical trials with corticosteroids added to the antibiotic regimen. Besides attenuation of the TNF-levels one of the proposed effects of corticosteroids involves the interference of neutrophil migration. Both for meningitis due to *H. influenza type b* as well as for pneumococcal meningitis this has become the standard of care. However, therapy with corticosteroids has many adverse effects.

As stated before, bacterial meningitis is not the only clinical situation where the influx of neutrophils has been associated with tissue damage (12 - 17). In serious brain injury (brain neurotrauma) and in ischemic events such as cerebrovascular ischemia, spinal cord injury/ischemia, myocardial infarction and intestinal ischemia, tissue reperfusion accompanied by neutrophil influx has repeatedly been correlated to tissue damage. Investigations into the role of neutrophils in non-infectious CNS damage have currently been intensified, since other therapeutical options in this field are very limited (18, 19).

Experimental animal models (20, 21) have been established to study various anti-inflammatory agents such as superoxide dismutase, transforming growth factor β, antibodies against adhesion molecules such as ICAM, caspase inhibitors, polysaccharides and cyclooxygenase inhibitors. Many of these strategies aim either directly at the reduction of the neutrophil influx or at (the release of) toxic neutrophil products into the CNS (22, 23). Even though the results look promising in animals, the effectiveness of these agents to reduce the migration of neutrophils into the CNS in the human situation remains to be established. Furthermore, there is considerable concern about potential side effects such as an adverse reaction to animal proteins (antibodies) and an increased susceptibility to infection through substantial interference with neutrophil function. Therefore, to our knowledge, the agents mentioned have not yet resulted in successful therapeutic tools that can be applied to the human situation. Thus, there is still an urgent need for new therapeutic means especially in CNS disease.

The present invention provides a novel means for suppressing neutrophil migration and thus a novel means for preventing damage resulting from neutrophil migration. The invention provides a proteinaceous substance derivable from ***Cryptococcus neoformans*** and capable of interfering with neutrophil migration in a mammalian host or a functional equivalent of such a proteinaceous substance for use as a pharmaceutical. Typically such proteinaceous substances in Cryptococcus itself are glycoproteins and more in particular so-called mannoproteins. However in other organisms functional equivalents (homologs) of these proteins may be found which have other characteristics but perform analogous functions as described herein. Also, based on the (3D) structure of the proteinaceous substances specifically disclosed herein, other molecules can be designed or produced that have at least one the same or similar function (in kind if not in amount). Also by mutating the specifically disclosed proteinaceous substances yet other functional equivalents may be prepared. These mutations are most easily performed through the genetic information underlying the proteinaceous substances of the present invention, which are of course also part of the present invention. Preferred embodiments of the invention are mannoproteins 1, 2 and 4 of ***Cryptococcus*** or functional fragments or derivatives thereof. In this respect functional fragments includes those fragments that have at least one similar or identical function of the original protein or carbohydrate moiety. Homologues are defined herein as proteins or carbohydrates having the same or similar function as the mannoproteins according to the invention but found in other organisms. Combinations of proteinaceous substances according to the invention may also be used. The afflictions for which the proteinaceous substances may be used are typically afflictions associated with an inflammatory response. Thus the invention includes the use of a proteinaceous substance or a functional equivalent thereof according to the invention in the preparation of a medicament for the treatment of diseases associated with inflammation, in particular for the treatment of a pathological condition or a disease involving neutrophil migration, more specifically a use whereby the inflammatory disease involves IL8, fMLP, PAF or C5a up-regulation. A preferred embodiment is a use according to the invention is the one whereby the inflammatory disease is meningitis, in particular bacterial meningitis. Other uses include those whereby the pathological condition is serious brain injury, Skin disease, Allergy, ARDS, Inflammatory bowel disease, Rheumatoid arthritis (or another autoimmune disease) or an ischemic event such as a myocardial infarct, cerebrovascular ischemia, other cardiovascular diseases, intestinal ischemia, cardiovascular surgery, pulmonary ischemia or skeletal muscular ischemia.
The invention further provides a pharmaceutical composition comprising a proteinaceous substance or a functional equivalent thereof according to the invention together with a suitable means for administration. Typically the administration route will be parenteral. Typically these compositions may comprise further therapeutic agents such as an anti-inflammatory drug, a corticosteroid, or an antibiotic agent.

### Detailed description.

In a particular preferred embodiment the invention provides the use of mannoproteins or a functional equivalent thereof in the preparation of a medicament for the treatment of a pathological condition or a disease involving neutrophil migration and in this sense as an anti-inflammatory agent.

In inflammation, soluble factors called chemokines have been identified which attract various subtypes of leukocytes. The chemokine IL8 is a potent chemoattractant for neutrophils (24). As expected, patients with bacterial meningitis have high levels of IL8 as well as an increased number of neutrophils in their CSF. We recently described that patients who suffer from a fungal meningitis caused by *Cryptococcus neoformans* whose CSF typically contains very little leukocytes, had high levels of IL8 in their CSF (25). Therefore, in cryptococcal meningitis, an agent must be present that interferes with neutrophil migration towards IL8. Cryptococcal polysaccharides have been shown to interfere with neutrophil movement (26 - 29). We and others in literature initially focused on the fungal capsular polysaccharide glucuronoxylomannan (GXM) as the cryptococcal agent responsible for inhibition of neutrophil migration. Patients with cryptococcal infection have high titers of GXM in both serum and CSF during infection (30, 31). We demonstrated *in vitro* that GXM also interferes with neutrophil migration (chemotaxis) towards IL-8 if GXM is added directly to the leukocytes (25). We also demonstrated (32) that a low CSF leukocyte cell count in patients with cryptococcal meningitis shows a significant inverse correlation to a high GXM titer in serum (relative to the GXM titer in CSF). In a rabbit model for pneumococcal meningitis we have recently shown that intravenous administration of GXM not only delays entry of leukocytes (polymorphonuclear cells, PMN) into the CSF and brain, but more importantly reduces TNFa levels in the CSF and protects the brains of these rabbits against tissue damage in comparison to control animals. The exact mechanism how GXM interferes with neutrophil migration in cryptococcosis is not known. Interestingly, in the second part of 1999, we discovered that another class of cryptococcal proteins (33), the mannoproteins (MP) are even more powerful than GXM in reducing neutrophil migration. We compared GXM and three different MP's (MP1, MP2 and MP4) for interference with different steps involved in transendothelial migration of PMN. 1.] MP inhibits migration of PMN towards IL8 (34), fMLP, C5a and PAF. 2.] GXM and MP were shown (35) to cause shedding of L-selectin (CD62L; the specific targeting of neutrophils to venules in inflamed tissue involves CD62L binding to E-and P-selectin on endothelial cells). 3.] GXM and MP caused upregulation of CDllb and CD18 (Mac-1). 4.] GXM and MP have intrinsic chemoattractive capacity. 5.] GXM and MP cause Ca-fluxes in leukocytes. In all assays MP turned out to be much more active than GXM; this holds true especially for MP4-which could be used at 20- to 100-fold lower concentrations than GXM while still causing higher absolute stimulation levels (CD11b/18 upregulation, chemotaxis, Ca-flux) or repression levels (inhibition of chemotaxis levels, CD62L shedding). Furthermore, we know that MP can be easily purified (36) and is not toxic; patients with cryptococcal meningitis can display relatively mild symptoms for a prolonged period of time despite the presence of MP in blood and CSF. Therefore, MP is a potent therapeutic agent. Off course functional equivalents of MP4 will be capable of interfering with neutrophil migration. Since the protein and carbohydrate compositions of MP1, MP2 and MP4 (the most potent mannoprotein) closely resemble each other, they are placed here together as "MP". Thus MP1, MP2, MP4 and functional equivalents thereof can be applied in the treatment of diseases or pathological conditions involving neutrophil migration. Typically such diseases include infections and brain neurotrauma were neutrophils invade the tissue, diseases with damage due to reperfusion ischemia were neutrophils are attracted and cause secondary damage, intensive care patients with ARDS and other inflammatory diseases (rheumatoid arthritis and inflammatory bowel disease). MP will be especially useful in diseases involving IL8 regulation. Examples thereof are brain inflammatory disease such as meningitis - in particular bacterial meningitis-, neurotrauma (brain, spinal cord), Lung disease (ARDS and COPD), Cardiovascular diseases (arteriosclerosis), Allergic skin diseases (Contact dermatitis, psoriasis) autoimmune diseases or the protection of the brain against toxic medication administered systemically (e.g. anti-cancer drugs). Other important applications will include the treatment of pathological conditions such as brain injury or ischemic events (ischemia - reperfusion after cardiopulmonary bypass surgery or other forms of cardiac surgery).

In a further embodiment the invention provides a pharmaceutical composition comprising mannoproteins 1,2 and 4 or functional equivalents thereof together with a suitable means for administration. Typically such a composition will be given systematically. The dose of MP necessary to prevent neutrophil migration may vary, but will generally be between 250 pg/kg and 10 mg/kg, preferably between 1 mg/kg and 5 mg/kg. The composition may of course contain other drugs for the treatment of diseases involving neutrophil migration, such as anti-inflammatory drugs, corticosteroids or an antibiotic agent.

The invention will be explained in more detail in the following experimental part.

Isolation and characterization of MP.

Mannoproteins were purified from the supernatant of an acapsular mutant of C. neoformans, CAP 67 (E.S.Jacobson, Medical College of Virginia) to avoid contamination with GXM. Cryptococcal cultures were grown for 5 days in RPMI supplemented with gentamycin to prevent bacterial contamination. Supernatants were harvested by centrifugation and directly applied to a concanavalin A column equilibrated in buffer A containing 10mM Tris pH 7.2, 500mM NaCl, 1mM MgCl2 and 1 mM CaCl2. The column was washed with buffer A and stepwise eluted with α-methyl-D-mannose pyranoside in PBS. MP1 and -2 eluted at 200 mM αmDm, MP4 eluted at 400 mM. MP1 and MP2 where further fractionated by anion exchange (DEAE, mono Q) chromatography. Purified MP is finally recovered by lyophilization. Samples are analyzed by various methods to prove that none of the purified mannoproteins contains constituents other than those known to occur in MP (36):

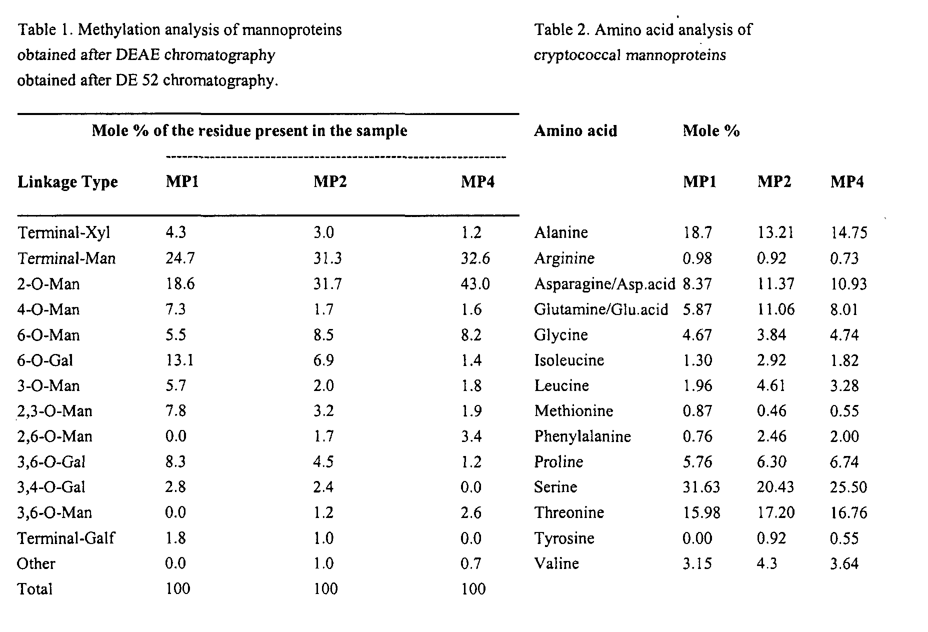

### References

1. Schlech WFI, Ward JI, Band JD et al. Bacterial meningitis in the US, 1978 through 1981. The national bacterial meningitis surveillance study. JAMA 1985; 253:1749-54.
2. Durand ML, Calderwood SB, Weber DJ et al. Acute bacterial meningitis in adults: a review of 493 episodes. N.Engl.J.Med. 1993; 328:21-8.
3. Pfister HW, Fontana A, Tauber MG, Tomasz A and Scheld WM. Mechanisms of brain injury in bacterial meningitis: workshop summary
4. Spellerberg B and Tuomanen EI. The pathophysiology of pneumococcal meningitis. Ann.Med. 1994; 26:411-8.
5. Hartl R, Medary MB, Ruge M, Arfors KE and Ghajar J. Early white blood cell dynamics after traumatic brain injury: effects on the cerebral microcirculation. J.Cereb.Blood Flow Metab. 1997; 17:1210-20.
6. Hallenbeck JM. Cytokines, macrophages and leukocytes in brain ischemia. Neurology 1997; 49:S5-9.
7. Winquist RJ and Kerr S. Cerebral ischemia-reperfusion injury and adhesion. Neurology 1997; 49:S23-6.
8. Yanada K, Camarata PJ, Spellman SR et al. Antagonism of leukocyte adherence by synthetic fibronectin peptide V in a rat model of transient focal cerebral ischemia. Neurosurgery 1997; 40:557-63.
9. Tuomanen EI, Saukkonen K, Sande S, Cioffe C and Wright SD. Reduction of inflammation, tissue damage and mortality in bacterial meningitis in rabbits treated with monoclonal antibodies against adhesion-promoting receptors of leukocytes. J.Exp.Med. 1989; 170:959-69.
10. Saez Llorens X, Jafari HS, Severien et al. Enhanced attenuation of meningeal inflammation and brain edema by concomitant administration of anti-CD18 monoclonal antibodies and dexamethasone in experimental Homophiles meningitis. J.Clin.Invest. 1991; 88:2003-11.
11. Quagliarello VJ and Scheld WM. Treatment of bacterial meningitis. N.Engl. J. Med. 1997; 336:708-16.
12. Korthuis RJ, Anderson DC and Granger DN. Role of neutrophil-endothelial cell adhesion in inflammatory disorders. J.Crit.Care 1994; 9: 47-71.
13. Tanaka M, Brooks SE, Richard VJ et al. Effect of anti-CD18 antibody on myocardial neutrophil accumulation and infarct size after ischemia and reperfusion in dogs. Circulation 1993; 87:526-35.
14. Schoenberg MH, Poch B, Younes M et al. Involvement of neutrophils in postischemic damage to the small-intestine. Gut 1991; 32:905-12.
15. Kubes P, Hunter J and Granger DN. Ischemia/reperfusion-induced feline intestinal dysfunction: importance of granulocyte recruitment. Gastroenterology 1992; 103:807-12.
16. Sekido N, Mukaida N, Harada A et al. Prevention of lung reperfusion injury in rabbits by a monoclonal antibody against interleukin-8. Nature 1993; 365:654-7.
17. Petrasek PF, Liauw S, Romaschin AD and Walker PM. Salvage of postischemic skeletal muscle by monoclonal antibody blockade of neutrophil adhesion molecule CD18. J.Surg.Res. 1994; 56:5-12.
18. Clark WM and Zivin JA. Anti-leukocyte adhesion therapy: preclinical trials and combination therapy. Neurology 1997; 49:S32-8.
19. Tuomanen E. Adjunctive therapy of experimental meningitis: agents other than steroids. Antibiot.Chemother. 1992; 45:184-91.
20. Ley K, Cerrito M and Arfors KE. Sulfated polysaccharides inhibit leukocyte rolling in rabbit mesentery venules. Am.J.Physiol. 1991; 260:H1667-73.
21. Ley K, Linnemann G, Meinen M, Stoolman L and Gaehtgens P. Fucoidin, but not yeast polyphosphomannan PPME, inhibits leukocyte rolling in venules of the rat mesentery. Blood 1993; 81:177-85.
22. Adams DH and Lloyd AR. Chemokines: leukocyte recruitment and activation cytokines. Lancet 1997; 349:490-5.
23. Taub DD. Chemokine-leukocyte interactions: the voodoo that they do so well. Cytokine Growth Factor Reviews 1996; 7:355-76.
24. Bell MD, Taub DD and Perry VH. Overriding the brain's intrinsic resistance to leukocyte recruitment with intraparenchymal injections of recombinant chemokines. Neuroscience 1996; 74:283-92.
25. Chaka WS, Heyderman R, Gangaidzo I et al. Cytokine profiles in CSF of HIV-infected patients with cryptococcal meningitis: no leukocytosis despite high IL-8 levels. J.Infect.Dis. 1997; 176:1633-6.
26. Drouhet E and Segretain G. Inhibition de la migration leukocytaire in vitro par un polyoside capsulaire de *Torulopsis (Cryptococcus)* neoformans. Ann.Inst.Pasteur 1951; 81:674-6.
27. Dong ZM and Murphy JW. Effects of two varieties of *Cryptococcus neoformans* cells and culture filtrate antigens on neutrophil locomotion. Infect.Immun. 1995; 63:2632-44.
28. Dong ZM and Murphy JW. Intravascular cryptococcal culture filtrate (CneF) and its major component, glucuronoxylomannan, are potent inhibitors of leukocyte accumulation. Infect.Immun. 1995; 63:770-8.
29. Dong ZM and Murphy JW. Mobility of human neutrophils in response to *Cryptococcus neoformans* cells, culture filtrate antigen, and individual components of the antigen. Infect.Immun. 1993; 61:5067-77.
30. Mitchell TG and Perfect JR. Cryptococcosis in the era of AIDS - 100 years after the discovery of *Cryptococcus neoformans.* Clinical Microbiology Reviews 1995; 8:515-48.
31. Eng RHK, Bishburg E and Smith SM. Cryptococcal infections in patients with acquired immune deficiency syndrome. Am.J.Med. 1986; 81:19-23.
32. Lipovsky MM, van Elden LJR, Walenkamp AME, Dankert J and Hoepelman AIM. Does the capsular component glucuronoxylomannan of *Cryptococcus neoformans* impair transendothelial migration of leukocytes in patients with cryptococcal meningitis? J.Inf.Dis. 1998; 178:1231-32
33. Cherniak R and Sundstrom JB. Polysaccharide antigens of the capsule of *Cryptococcus neoformans.* Infect.Immun. 1994; 62:1507-12.
34. Lipovsky MM, Gekker G, Hu S, Ehrlich LC, Hoepelman AIM and Peterson PK. Cryptococcal glucuronoxylomannan induces interleukin 8 (IL8) production by human microglia but inhibits neutrophil migration towards IL8. J.Infect.Dis. 1998; 177:260-3.
35. Dong ZM and Murphy JW. Cryptococcal polysaccharides induce L-selectin shedding and tumor necrosis factor receptor loss from the surface of human neutrophils. J.Clin.Invest. 1996; 97:689-98.
36. Walenkamp AME, Chaka WS, Verheul AFM, Vaishnav W, Cherniak R, Coenjaerts FEJ and Hoepelman IM. *Cryptococcus neoformans* and its cell wall components induce similar cytokine profiles in human peripheral blood mononuclear cells despite differences in structure. FEMS Imm and Med. Microbiology 1999; 26:309-18

## Claims

1. A proteinaceous substance derivable from ***Cryptococcus neoformans*** and capable of interfering with neutrophil migration in a mammalian host or a functional equivalent thereof for use as a pharmaceutical.

2. A proteinaceous substance for use according to claim 1 which is a glycoprotein.

3. A proteinaceous substance for use according to claim 1 or 2, which is a mannoprotein or a functional equivalent thereof.

4. A proteinaceous substance for use according to claim 1-3, which is mannoprotein 1, 2 or 4, or a functional fragment, and/or a functional homologue and/or a combination thereof.

5. Use of a proteinaceous substance or a functional equivalent thereof according to any one of claims 1-4, in the preparation of a medicament for the treatment of diseases associated with inflammation.

6. Use of a proteinaceous substance or a functional equivalent thereof according to any one of claims 1-4, in the preparation of a medicament for the treatment of a pathological condition or a disease involving neutrophil migration.

7. Use according to claim 5 or 6, whereby the disease is an infection or an inflammatory disease.

8. Use according to claim 7 whereby the inflammatory disease involves IL8, fMLP, PAF or C5a up-regulation.

9. Use according to anyone of claims 5-8, whereby the inflammatory disease is meningitis, in particular bacterial meningitis.

10. Use according to any one of the aforegoing claims whereby the pathological condition is serious brain injury, Skin disease, Allergy, ARDS, Inflammatory bowel disease, Rheumatoid arthritis (or another autoimmune disease) or an ischemic event.

11. Use according to claim 10 whereby the ischemic event is a myocardial infarct, cerebrovascular ischemia, other cardiovascular diseases, intestinal ischemia, cardiovascular surgery, pulmonary ischemia or skeletal muscular ischemia.

12. A pharmaceutical composition comprising a proteinaceous substance or a functional equivalent thereof according to any one of claims 1-4 together with a suitable means for administration.

13. A pharmaceutical composition according to claim 12, further comprising an anti-inflammatory drug.

14. A pharmaceutical composition according to claim 12 or 13, further comprising a corticosteroid.

15. A pharmaceutical composition according to any one of claims 12-14, further comprising an antibiotic agent.
